(19) Europäisches Patentamt
European Patent Office
Office européen des brevets

(11) Publication number: 0 219 229 B1

(12) EUROPEAN PATENT SPECIFICATION

(45) Date of publication of patent specification: 25.03.92 (51) Int. Cl.5: C07C 29/86

(21) Application number: 86307140.3

(22) Date of filing: 16.09.86

# (54) Improved process for recovery of alcohols from acid streams.

(30) Priority: 16.09.85 US 776387

(43) Date of publication of application:
22.04.87 Bulletin 87/17

(45) Publication of the grant of the patent:
25.03.92 Bulletin 92/13

(84) Designated Contracting States:
DE FR GB IT NL

(56) References cited:
GB-A- 493 884
GB-A- 506 473
US-A- 2 139 953
US-A- 3 489 779
US-A- 4 538 010

INDUSTRIAL AND ENGINEERING CHEMISTRY PROCESS DESIGN AND DEVELOPMENT, vol. 23, no. 1, January 1984; Curtis L. MUNSON et al, "Factors Influencing Solvent Selection for Extraction of Ethanol from Aqueous Solutions", pp. 109-115

(73) Proprietor: EXXON RESEARCH AND ENGINEERING COMPANY
P.O.Box 390, 180 Park Avenue
Florham Park, New Jersey 07932(US)

(72) Inventor: Diana, William Daniel
91 Ridgeview Drive
Belle Mead New Jersey(US)
Inventor: Yeh, Chuen Yuan
15 Stratford Circle
Edison New Jersey(US)

(74) Representative: Bawden, Peter Charles et al
Esso Chemical Research Centre PO Box 1
Abingdon Oxfordshire OX13 6BB(GB)

EP 0 219 229 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates generally to the recovery of alcohols from concentrated aqueous acid streams.

Large volumes of alcohols are produced annually by the catalytic hydration of olefins, in which the selected olefin feed is absorbed in a concentrated aqueous sulfuric acid stream to form the corresponding alcohol and alkyl ester of the sulfuric acid. For example, the absorption of butene to form sec-butanol and a sec-butyl ester of sulfuric acid can be illustrated by the following equation:

$$CH_3CH{=}CHCH_3 \underset{H_2O}{\overset{H_2SO_4}{\rightleftharpoons}} CH_3\underset{\displaystyle OH}{\underset{|}{C}}HCH_2CH_3 + CH_3\underset{\displaystyle OSO_3H}{\underset{|}{C}}HCH_2CH_3$$

Thereafter, water is admixed with the sulfuric acid stream withdrawn from the absorber to hydrolyze the ester and to facilitate alcohol recovery which is accomplished by stripping with steam. There is thereby produced a diluted sulfuric acid stream which must for economic reasons be treated to concentrate it with respect to its sulfuric acid content, after which it is recycled to the olefin absorption step.

The reconcentration of the diluted sulfuric acid stream is a very expensive and energy-intensive process step, and a method whereby the alcohol could be recovered from the sulfuric acid stream withdrawn from the absorber, which did not require such a reconcentration, would be highly desirable.

Processes have been proposed for alcohol recovery by extraction from such alcohol-containing sulfuric acid streams by use of benzene, chloroform, ether, carbon bisulfide and toluene (U.S. Patent 1,365,046); phenols, cresols, their homologues, ethers and phosphates (U.S. Patent 2,139,953); and saturated hydrocarbons (British Patent 493,884). British Patent 506,473 relates to a method for production of organic oxy-compounds from a mixture of two liquid phases containing the oxy-compound distributed between the two layers in which the layers are separated and then recontacted at a lower temperature to extract the oxy-compound from the more hydrophobic phase, e.g., a hydrocarbon phase.

However, such extraction methods are not commercially desirable, since the solvents extract very little alcohol if the acid strength of the alcohol-containing sulfuric acid stream is greater than about 55%. Dilution of the acid stream to improve the ability of these solvents to extract the alcohol is not economically practicable, since even more expense would be incurred in reconcentrating the resulting sulfuric acid (recovered after the extraction) prior to its being recycled to the olefin absorbing step.

C. L. Munson et al., Ind. Eng. Chem. Proc. Des. Dev., vol. 23, no. 1, pp. 109-115 (1984) (which is not admitted herein to be prior art) investigated equilibrium distribution coefficients and separation factors for extraction of ethanol from dilute aqueous solutions of the alcohol by a number of different solvents and solvent mixtures, including extraction solvents comprising neodecanoic acid and 2-ethyl hexanoic acid. An ethanol-water-extraction solvent phase is obtained and treated to dehydrate the ethanol, followed by fractionating the ethanol and solvent mixture. The dilute aqueous raffinate is separated from the extractor and treated, as by stripping, to separate extraction solvent dissolved in the raffinate.

U.S. Patent 3,489,779 to Esso Research and Engineering Company is directed to a process for purifying neo-carboxylic acids by treating the neo acid with concentrated sulfuric acid (85+ wt.% $H_2SO_4$) followed by treatment with a phase-separating agent, which can either comprise water or a paraffinic hydrocarbon. The function of the concentrated sulfuric acid is to convert olefinic and other impurities (e.g., Fe) into the corresponding sulfates. If water is added as the phase-separating agent it must be used in carefully controlled amounts, sufficient to liberate the neo-acid from its sulfuric acid complex but insufficient to hydrolyze the alkyl sulfates and other impurites formed in the first step. A water and neo-acid mixture, containing a lessened impurity level, is recovered. When a paraffinic hydrocarbon is used, a paraffin and neo-acid mixture is recovered as a separate liquid phase, and the sulfate impurities remain in the sulfuric acid phase. In the latter process alternative, the separated paraffin/neo-acid layer still contains 5-10 wt.% $H_2SO_4$ which must be removed.

According to the process of the present invention, alcohols are recovered from aqueous mixtures thereof with a concentrated strong acid by (a) contacting such alcohol-containing aqueous concentrated acid mixtures with a carboxylic acid extractant selected from the group consisting of acids of the formula $RCO_2H$ wherein R is a straight or branched-chain or cyclic alkyl of from 5 to 19 carbon atoms per molecule to form an extract phase containing the carboxylic acid in addition to the alcohol, and an aqueous raffinate phase depleted in the alcohol and containing entrained extractant, (b) recovering the alcohol from the extract

phase, and (c) contacting the aqueous raffinate phase with a organic extractive agent to separate at least a majority of the entrained carboxylic acid extraction solvent therefrom.

As disclosed in Serial Number 650,875, filed September 17, 1984 and published as European Publication No. 0175443 on March 26, 1986 (the disclosure of which is hereby incorporated by reference), it has been surprisingly found that the above extraction solvents efficiently remove alcohol from concentrated aqueous strong acid solutions thereof and permit recovery of this alcohol without substantial dilution of the aqueous strong acid. The extraction solvents of this invention permit only minor amounts of water to pass into the extraction solvent phase so that alcohol can be recovered substantially free of water. However, the recovered strong aqueous acid raffinate, depleted in alcohol, has been unexpectedly found to contain (entrained and/or solubilized therein) small amounts of the carboxylic acid extraction solvent. The present inventors have discovered that the carboxylic acid extraction solvent can be quickly and economically separated from such raffinates by contacting the raffinate with a organic extractive agent. The carboxylic acid partitions efficiently into the organic extractive agent and permits a strong acid raffinate, freed of substantial contamination by the carboxylic acid, to be recovered. The thus treated strong acid raffinate can then be readily recycled, for example, directly to an olefin absorption process for absorption of additional olefin.

Figure 1 is a diagrammatic illustration of a prior art process for indirect hydration of olefins using sulfuric acid, dilution of the sulfuric acid extract and stream stripping of the diluted acid for recovery of the alcohol vapors.

Figure 2 is a diagrammatic illustration of one embodiment of the process of this invention.

The prior art method of producing alcohols by hydrating the corresponding olefin can be illustrated by reference to Figure 1. An olefin, for example an aliphatic olefin having from 2 to 8, and more typically from 2 to 4, carbon atoms per molecule (e.g., ethylene, propylene, or 1- or 2-butene) is fed as a gas or liquid via line 2 to an absorber 10 wherein it is contacted with and absorbed (at least in part) by a concentrated aqueous strong acid stream introduced via line 6, to form the corresponding alcohol and alkyl ester of the strong acid.

The olefins to be hydrated can be obtained from any available source, such as the destructive distillation of carbonaceous materials, but particularly from the cracking of petroleum hydrocarbons such as is practiced in the petroleum refining of mineral oils. The olefin can also be conventionally obtained by careful fractionation of cracked petroleum gases and is preferably substantially free of higher unsaturates, particularly diolefins such as butadiene, etc. Illustrative of olefins which are employed are lower branched and straight-chain alkenes (i.e., alkenes of 2 to 6 carbon atoms), such as ethylene, propylene, the butylenes and the like.

The strong acid used to absorb the olefin in absorber 10 (also termed "olefin hydration acid") will generally comprise a strong organic or inorganic acid which is miscible with water and which is characterized by dissociation constants ("pK" values) in aqueous solutions of less than about 3.5. Examples of suitable inorganic olefin hydration acids are hydrofluoric acid, hydroiodic acid, hydrochloric acid, orthophosphoric acid, phosphorous acid, perchloric acid, sulfuric acid and the like. Sulfuric acid is especially preferred. Examples of suitable organic olefin hydration acids are chloroacetic acid, benzene sulfonic acid and the like. For convenience, the following discussion will be directed to the use of sulfuric acid, although it will be understood that any of the above strong acids could also be employed.

The aqueous strong acid stream 6 which is used to absorb the selected olefin feed is a concentrated acid stream whose precise acid concentration will vary depending on the olefin which is employed, the strong acid selected, the temperatures of reaction and other conditions. For example, when sulfuric acid is used as the strong acid, stream 6 will generally contain from about 45 to 99 % acid strength sulfuric acid for hydration of propylene and from about 55 to 85 % acid strength sulfuric acid for reaction with butylene or higher olefin feeds.

The temperature and pressure employed in absorber 10 generally also varies depending on the olefin, the acid concentration and other factors. Generally, a temperature of from about 20 to 150 °C is used, and the pressure is sufficient to maintain the desired liquid phases in the absorber. Typically, for example, propylene is absorbed from a gas phase at a temperature of from about 90 to 150 °C, and at a pressure of from about 100-500 psig.

As illustrated, the olefin and sulfuric acid streams are contacted in a counter-current fashion with the sulfuric acid stream being introduced into the upper portion of the absorber 10. Unabsorbed gases are withdrawn from the upper portion of absorber 10 via conduit 7 and can be recycled, if desired, to conduit 2 or can be subjected to conventional scrubbing/washing treatment, as with caustic solutions. A product stream, comprising a sulfuric acid solution of the alcohol (herein termed the "absorber product stream"), is withdrawn via line 4 from the lower portion of absorber 10. The absorber product stream can also contain

the alkyl ester corresponding to the selected olefin, e.g., monoethyl sulfate when ethylene is the olefin, and mono-isopropyl sulfate in the case of propylene hydration. The concentration of the alkyl ester is stream 4 can vary widely, and is generally from 15 to 30 wt.% of the total alkyl ester (mono- and di-alkyl ester) in the case of lower alkenes (e.g., propylene and butylene) absorption.

In the second step of the hydration process, water is conventionally added via line 12 to the absorber product stream 4 for hydrolysis of any alkyl ester and to form additional quantities of the corresponding alcohol, e.g., isopropanol from mono- or di-(isopropyl) sulfate. The manner in which the water and absorber product stream are contacted varies, and the art employs a variety of such methods, including (1) in-line addition of water (as illustrated), with a provision for a suitable length of conduit to provide adequate mixing and reaction time, and (2) contacting of the absorber product stream and water in a separate reaction vessel with agitation (not shown).

The amount of water which is added to the absorber product stream also varies widely. Generally, sufficient water is added in order to reduce the acid strength to from 45% to 55% sulfuric acid. These reduced acid strengths are desired to permit subsequent recovery of the alcohol by steam stripping. Typically, from about 0.2 to 0.5 parts by weight of water is added per part by weight of the absorber product stream.

The diluted stream thus formed generally contains from about 45 to 55 wt.% sulfuric acid, and is then passed via line 4 to distillation column 20, herein termed the "alcohol generator," wherein crude alcohol is recovered as an overhead product via line 18 by steam stripping. The overhead alcohol product can then be passed to further conventional processing to produce alcohol of the required purity.

A bottoms product is withdrawn from alcohol generator 20 via line 28 and comprises a sulfuric acid stream which generally contains from about 40 to 55 wt.% sulfuric acid.

In conventional processes, the alcohol generator bottoms 28 are passed directly to another distillation column 30, hereinafter term the "acid concentrator", wherein this acid stream is distilled (e.g., by use of a steam heat exchanger 34 and reboiled stream 37) for removal of water as overhead 32 and to form a second bottoms product 38 comprising a reconcentrated acid stream. These concentrated bottoms are generally cooled in cooler 40 and passed to storage tank 50 for ultimate recycle to the absorption step 10, with addition of make-up acid 5, as required.

Referring to Figure 2, a concentrated aqueous strong acid alcohol-containing solution 104 (herein termed the "acid/alcohol feedstream" is contacted with an effective amount of an extraction solvent 166 selected from the group consisting of alicyclic and acyclic alkyl carboxylic acids having from 6 to 20 carbon atoms per molecule. The carboxylic acids which are employed as extraction solvents 166 in the process of this invention therefore comprise at least one member selected from the group consisting of carboxylic acids of the formula:

$$R-\overset{\overset{O}{\|}}{C}-OH \qquad (I)$$

wherein R is an alicyclic or acyclic alkyl group having from 5 to 19 carbon atoms. When "R" is acyclic alkyl, the alkyl group can be straight or branched chain. The "R" group can be substitued with nonreactive groups such as fluoro and chloro. Examples of such "R" groups are pentyl, hexyl, decyl, dodecyl, tetradecyl, undecyl, 2-ethylhexyl, cyclohexyl, cyclooctyl and fluoro- and chloro- substituted derivatives of the foregoing.

A preferred class of carboxylic acid extraction solvents for use in this invention comprise saturated acids, and especially saturated hindered acids wherein the carboxyl groups are sterically blocked or hindered. The discussion of steric hindrance may be found in Newman, Steric Effects in Organic Chemistry, 1956, pp. 204-207. Generally, steric hindrance results from the presence of tertiary or quaternary alpha, beta or gamma carbon atoms in the acid, increasing substitution leading to increased hindrance. Steric hindrance has been observed to tend to prevent esterification of the carboxylic acid with the alcohol.

A class of particularly preferred sterically hindered carboxylic acids are the neo acids ("neo" is used to denote a carbon atom that is bonded to four other carbon atoms, e.g., as in neo-hexanoic acid, i.e., 2,2-dimethyl butanoic acid). Thus, the preferred neo-acids can be represented by members selected from the group consisting of acids having the formula (II):

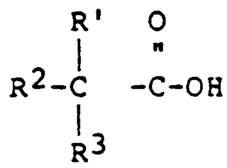

(II)

wherein $R^1$, $R^2$ and $R^3$ are the same or different and are independently selected from the group consisting of cyclic and alicyclic alkyl of from 1 to 16 carbon atoms, with the proviso that the neo acid contains a total of from 6 to 20 carbon atoms per molecule, and preferably from 7 to 15 carbon atoms per molecule. The alicyclic alkyl groups can be straight or branched. A preferred class of neo acids are those wherein, referring to formula II above, $R^1$ and $R^2$ are preferably each alkyl radicals having from 1 to 3 carbon atoms per radical and $R^3$ is preferably alkyl having from 1 to 6 carbon atoms, provided that the neo-acid has a total of at least 6, and preferably at least 7, carbon atoms per molecule.

Some typical examples of neo acids are 2,2-dimethyl butanoic acid; alpha-dimethyl cyclohexyl acetic acid; alpha,alpha-dimethyl octanoic acid; 1-methyl-4-propylcyclohexane-1-carboxylic acid; and the like.

Commercial neo-acids which comprise a mixture of isomers, all with the neo structure (formula II), such as neo-heptanoic acid, neo-octanoic acid, neononanoic acid, neo-decanoic acid and neo-tridecanoic acid, are especially useful and preferred in the process of this invention. The preparation and properties of the commercial carboxylic acids are described in N. Fefer and A. Rutkouski, J. Am. Oil Chemists' Soc., 45, 5 (1968). Neo acids can also be prepared by the well-known Koch process from carbon monoxide, water and type II, III, IV or V olefins as described by H. Koch in Brenntstaff Chem.,36, 321 (1955). Further details on methods for making neo acids can be found in British Patent 998,974 and U.S. Patent 3,349,107, all of which are hereby incorporated by reference. Neo acids are often made from branched chain olefin feedstocks which are random isomeric mixtures in regard to the position of the olefinic bond. These acids are thus random isomeric mixtures of neo acids. These neo acids are suitable in their isomeric forms, or any suitable mixtures thereof may be employed.

The carboxylic acid extraction solvent may be used alone or as mixtures with a cosolvent for the selected alcohol, such as $C_{10}$ to $C_{20}$ paraffinic hydrocarbon, a $C_7$ to $C_{12}$ aromatic hydrocarbon, or a $C_1$ to $C_{10}$ alkyl ester of a $C_4$ to $C_{15}$ monocarboxyic acid. When used, such additional cosolvents will be generally employed in such mixtures in an amount of less than about 70 wt.% (and preferably less than 50 wt.%) of the carboxylic acid/cosolvent mixture. In particular, it has been observed that the carboxylic acid extraction solvents of this invention, when contacted with alcohol and concentrated aqueous acid, will form the corresponding carboxylic ester of the alcohol, albeit at a rate which varies depending on the particular carboxylic acid extraction solvent selected for use. For example, steric hindrance in the carboxylic acid, especially the neo structures (Structure II) will tend to reduce the rate and equilibrium constant of the esterification reaction. Eventually, the thus-formed ester of the carboxylic acid extraction solvent will build up to approximately a steady-state concentration in the continuous process in which the carboxylic acid extraction solvent is repeatedly recycled to extract additional quantities of alcohol from the alcohol-containing concentrated aqueous acid feedstream (as will be described in more detail below).

The selected carboxylic acid extraction solvent of this invention may be contacted with the acid/alcohol feedstream in extraction zone 160 in any convenient manner, including continuous, semi-continuous or batchwise operations, in a single or in multiple extraction stages. The concentration of the strong acid (e.g., sulfuric acid) in acid/alcohol feedstreams 104 (herein termed the "acid strength" or "A.S.") to be so contacted, can vary widely and will typically range from about 40 to 80 wt.% sulfuric acid, preferably from about 50 to 65 wt.% sulfuric acid when a neo acid is employed as the extracting solvent. As used herein, the acid strength in the acid/alcohol feedstream is defined herein on an organic free basis, as follows (using $H_2SO_4$ as the acid for purposes of illustration)

$$A.S. = \frac{W_1 + \left[\frac{M_1 \times W_4}{M_1 + M_5}\right]}{W_1 + W_2 + \left[\frac{18W_3}{M_3}\right] + \left[\frac{M_1 \times W_4}{M_1 + M_5}\right]} \times 100$$

wherein $W_1$ is the weight of the strong acid, $W_2$ is the weight of $H_2O$, $W_3$ is the weight of alcohol, $W_4$ is the weight of the mono alkyl ester of the strong acid, $M_3$ is the molecular weight of the alcohol, $M_1$ is the molecular weight of the strong acid, and $M_5$ is the molecular weight of the olefin. Also, the concentrations of the alcohol and alkyl ester in the stream 104 can vary widely, and the saturated monoalcohol concentration will generally range from about 5 to 50 wt.% and preferably from 10 to 40 wt.% and the saturated alcohol alkyl ester of the strong acid will generally range from about 1 to 15 wt.%, and preferably from about 1 to 5 wt.%, of total alkyl ester (mono- and di-alkyl ester).

The amount of carboxylic acid extraction solvent which is used to contact the acid/alcohol feedstream can also vary widely. Preferably, the carboxylic acid extraction solvent and the acid/alcohol feedstream are contacted in zone 160 in a ratio of from about 0.3 to 5 parts by weight of the carboxylic acid per part by weight of the acid/alcohol feedstream.

The temperature and pressure in zone 160 in which such extraction is performed using the carboxylic acid solvents of this invention is not critical, and will vary widely depending upon the particular carboxylic acid solvent employed, the degree of efficiency or removal desired for the alcohol, phase settling time desired, and other factors. Generally, however, temperatures within the range from about 25 to 100 °C, preferably from about 25 to 80 °C, can be used, and the pressure can be atmospheric, subatmospheric ro superatmospheric, with pressures of from about 2 to 100 psig being typically suitable. Similarly, the time for the contacting of the selected carboxylic acid solvent and the acid/alcohol feedstream is not critical and can vary widely, but will typically fall within the range of from about 0.1 to 6 hours.

Finally, the process of this invention can employ as extraction zone 160, in which the extraction with carboxylic acid solvent is carried out, any of the conventional equipment which are employed for liquid-liquid extractions. For example, the solvent and the acid/alcohol feedstream can be introduced as a combined stream into a stirred tank, and the resulting liquids passed to a settling drum from which the light phase (alcohol solvent phase) and the heavy phase (lean acid/alcohol phase) can be recovered. Alternatively, the contacting zone can comprise a static plate extraction column (either packed or trayed column), a reciprocating plate column (such as the KARR® column), stirred columns (such as YORK-SCHIEBEL® columns) and the like.

In the process of this invention, water may be added to the acid.alcohol feedstream 104 in order to provide the water of hydration for formation of the selected alcohol from alkyl ester corresponding to the alcohol. This water can be introduced via stream 112 into the conduit carrying the acid/alcohol feedstream 104 as illustrated, or the water can be introduced into zone 160 itself. Generally, form about 0.04 to 0.4 parts by weight of water are added per part by weight of the acid/alcohol feedstream.

An alcohol-rich carboxylic acid extract stream 162 can be thus separated and recovered as the light phase from extraction zone 160, either directly from zone 160 (as illustrated) or following treatment of a two-phase mixture removed from the contacting zone to a conventional phase separation vessel. This alcohol-rich carboxylic extract 162 generally contains from about 5 to 20 wt.% alcohol and from about 95 to 45 wt.% of the carboxylic acid solvent. The alcohol-rich carboxylic acid extract 162 will only contain a minor proportion of water, preferably less than about 2 wt.% water, more preferably less than about 1 wt.% water, and most preferably less than about 0.3 wt.% water. In addition, it has been found that the carboxylic acid extraction solvents of this invention permit recovery of such an alcohol-rich stream without, at the same time, contamination with significant amounts of the strong acid or alkyl ester of the strong acid. Generally, the alcohol-rich carboxylic acid extract 162 recovered from the contacting zone contains less than about 1 wt.%, most preferably less than about 0.3 wt.%, of such alkyl esters and is substantially free of the strong acid, that is, contains less than 0.1 wt.% and most preferably less than 0.01 wt.%, of the strong acid (e.g., $H_2SO_4$).

A second phase 168, comprising the aqueous stong acid raffinate, can also be recovered, again either directly or indirectly, from the extraction zone 160 as the heavy phase and comprises a mixture of water,

strong acid, alkyl moieties (either as the mono-ester or di-ester of the strong acid or the alcohol, or mixtures thereof), and minor amounts of the carboxylic acid extraction solvent, and is enriched in the strong acid. Generally, this strong acid phase 168 separated from the extraction zone 160 will contain strong acids in an acid strength of at least 1 wt.%, and perferably from about 2 to 10 wt.% greater than the acid strength of the strong acid in the diluted acid/alcohol feedstream 117 passed to extraction zone 160.

The quantity of the carboxylic acid extraction solvent contained in the aqueous strong acid raffinate phase 168 will be from about 0.1 to 2.0 wt.%, of the aqueous strong acid raffinate. The aqueous strong acid raffinate 168, according to the process of this invention, is contacted with a liquid-organic compound 152 and the resulting mixture is passed into a liquid-liquid separation zone 130 wherein separate liquid phases are allowed to form, e.g., as by settling. If desired, the organic extractive agent can be introduced into conduit 168 via conduit 152 (as shown in Figure 2). Alternatively, a part or all of the organic extractive agent can be added directly to the settling drum 130. A heavier liquid phase can be then recovered via conduit 156 and comprises an aqueous strong acid stream depleted in the carboxylic acid extraction solvent. An upper liquid phase can be withdrawn via conduit 154 and comprises the organic extractive agent phase containing the separated carboxylic acid extraction solvent.

The recovered aqueous strong acid phase 156, now depleted in both alcohol and the carboxylic acid extraction solvent, can be recycled to an olefin absorbing zone 110, after addition of make-up strong acid 105, if required and after cooling in cooler 140 and intermediate storage in tankage 150, if desired.

The organic extractive agent 152 thus admixed with the aqueous strong acid raffinate 168 can comprise any organic compound (1) having a limited solubility in the aqueous strong acid and (2) in which the neo-acid contaminant is soluble under the conditions in which the organic extractive agent and stream 168 are contacted. Preferably, the organic extractive agent has a solubility at such conditions of less than 5 wt.%, more preferably of less than 1 wt.%, in the strong acid 168 containing the neo-acid contaminant. The organic extractive agent can therefore comprise members selected from the group consisting of alkanes of 2 to 12 carbon atoms, alkenes of 2 to 12 carbon atoms, cycloalkanes of 3 to 12 carbon atoms, and di-alkyl ethers of from 3 to 20 carbon atoms and mixtures thereof. Examples of the foregoing organic extractive agent s include butylene, propene, propane, butane, octane, octenes, pentene, pentane, di(sec-butyl) ether, di(pentyl) ether, di(isopropyl) ether, hexane, heptene, hexene, heptane, nonane, nonene, decene, decane, undecane, undecene, dodecane, dodecene, and the like.

Most preferably, the selected organic extractive agent will correspond to an organic compound which is indicative to the overall process for producing the alcohol which was sought to be recovered by the neo-acid extraction step. For example, in the production of isopropyl alcohol, it is preferred that the organic extractive agent s comprise propylene or diisopropyl ether; for production of secondary butyl alcohol, n-butenes or secondary butyl ether; for production of tertiary butyl alcohol, isobutylene or di-tert-butyl ether; Of course, mixtures of such organic extractive agents can also be used, such as mixtures of paraffins and olefinically unsaturated compounds (e.g., a liquid stream containing butane and butenes).

The particular organic extractive agent which is selected will to a large measure depend on the capacity of the organic extractive agent for the neo-acid contaminant, and also on how the separated organic extractive agent can be employed in the overall process. Thus, whereas capacity for neo-decanoic acid of octenes is greater than that of butenes, on equal mass treat ratios, butenes are generally preferred in the overall process in which secondary butyl alcohol is produced by absorption of butenes in the sulfuric acid absorption step.

Most preferably, the organic extractive agent is one which possesses a normal boiling point which is less than the normal boiling point of the neo-acid contaminant, to provide ease of separation of these two materials following removal from settling drum 130. Therefore, liquid organic solvents having a normal boiling point of about 100 to 200 °C lower than the normal boiling point of the neo-acid contaminant are most preferred.

The quantity of the organic extractive agent contacted with second phase 168 can vary widely, and will generally range from about 5 to 60 parts by weight, preferably from about 10 to 50 parts by weight and most preferably from about 20 to 40, of the organic extractive agent per 100 parts of the second phase 168. Similarly, the temperatures and pressures employed in settling drum 130 can vary widely depending on such factors as the quantity of the carboxylic acid extraction solvent in second phase 168, the particular liquid organic solvent employed and other factors. Generally, however, temperatures of from about 30 to 150 °C (and preferably from about 40 to 70 °C) and pressures of from about 15 to 200 psig (10546 to 140614 Pa) and more preferably from about 30 to 60 psig (21092 to 42184 Pa) will be suitable. The residence time of the liquid mixture of the aqueous strong acid raffinate and the selected organic extractive agent in settling zone 130 can vary widely but will generally range from about 0.1 to 5 hours (more preferably from about 0.2 to 2 hours). Preferably, the contacting of the liquid organic compound and second

phase 168, for removal of the carboxylic acid extraction solvent therefrom, is conducted for a time and under conditions sufficient to effect separation of at least a majority (i.e., at least about 50 wt.%) of the carboxylic acid extraction solvent initially present in the second phase 168, and more preferably from about 50 to about 80 wt.% or more of the such initially present amount of the carboxylic acid extraction solvent.

The upper liquid phase formed in settling drum 130 comprises the separated organic extractive agent and carboxylic acid extraction solvent and can be withdrawn from drum 130 via conduit 154. If desired, liquid 154 can be treated to separate the organic extractive agent and the carboxylic acid extraction solvent by any suitable means (e.g., by distillation) and the thus recovered carboxylic acid extraction solvent can be recycled to the extractor 160 (e.g., by introducing this recycled extraction solvent into conduit 166) for extraction of additional alcohol values in vessel 160. The thus-separated organic extractive agent can be returned via conduit 152. Alternatively, as in the case in which the selected organic extractive agent comprises the olefin which is desired for conversion into alcohol, the separated organic extractive agent can be passed to conduit 102 for absorption in absorber 110. Also, the organic extractive agent thus recovered can be employed in vessel 170 to assist in the stripping of extracted alcohol values from the carboxylic acid extract 165, as is described in more detail below.

Preferably, the extraction in absorber 160 is performed by continuously passing the acid/alcohol feedstream 117 and the carboxylic acid extraction solvent 166 to the extraction zone 160 countercurrently, and at flow rates such that the stong acid phase, that is the acid/alcohol feedstream 117, is the discontinuous phase and is introduced to the upper portion of the extraction tower 160, with the extraction solvent phase comprising the continuous phase. It has been found that such an embodiment greatly reduces the time required for phase separations to occur during the extraction.

The alcohol rich carboxylic acid extract 162 can then be treated for recovery of the alcohol therefrom and for recovery and separation of the carboxylic acid solvent for recycle, if desired to the extraction zone 160 in order to extract additional quantities of alcohol therein. The manner in which the alcohol is removed from this alcohol enriched phase can vary widely, and can include steam stripping or the use of the alcohol recovery methods described in co-pending Patent Number 4,538,010, granted August 27, 1985 (the disclosure of which is hereby incorporated by reference), which methods include distillation, flashing, or stripping using a substantially anhydrous gas which is inert to the alcohol and extraction solvent under stripping conditions, such as $N_2$, and olefins and paraffins of 2 to 8 carbons, e.g., butene, butane, and the like. For example, the separated alcohol-enriched carboxylic acid extract phase 162 can be heated (e.g., using heat exchanger 167) to a temperature of from about 0 to 200° C higher than the temperature employed in extraction zone 160, and the resulted heated alcohol-enriched stream 165 can be flashed into a vapor/liquid separation zone 170 (such as a suitable vapor/liquid separating drum) at a lower pressure in order to effect vaporization of the alcohol and thereby permit recovery of alcohol vapors 172 from this flashing zone. A lean solvent phase comprising the carboxylic acid extraction solvent can be withdrawn from flashing zone 170 via conduit 166 and recycled to extraction zone 160, after addition of make-up carboxylic acid extraction solvent 169, if required.

The conditions of temperature and pressure which are employed in flashing zone 170 for alcohol recovery will vary widely depending upon the alcohol to be recovered, the particular carboxylic acid extraction solvent which is used, and other factors. Generally, however, the flashing zone 170 should employ a temperature of from about 30 to 150° C, preferably from about 60 to 120° C, for recovery of alcohols of from 2 to 8 carbon atoms. Preferably, the carboxylic acid extraction solvent which is used in this invention, when such a flashing method of alcohol recovery is to be used, possesses a normal boiling point of at least 100°, more preferably at least 140°, greater than the normal boiling point of the alcohol in order to permit more efficient separation of the alcohol vapors 172 without, at the same time, contaminating the thus-separated vapors with significant quantities of the carboxylic acid solvent itself.

The conditions of pressure which are used in flashing zone 170 will, of course, have an effect on the necessary temperatures to recover the alcohol by flashing, but generally, the pressure in the flashing zone 172, will range from about 2 to 150 psig (1406 to 105460 Pa), and more preferably from about 5 to 50 psig (3515 to 35150 Pa).

By this flashing means, introduction of steam into the alcohol-carboxylic acid mixture is prevented. Such steam introduction is undesired since it has been observed that the carboxylic acid's affinity for water is greatly increased in the absence of sulfuric acid. Thus, if open steam distillation or steam stripping were used, while alcohol vapors could be stripped overhead, the bottoms stream from such a steam stripper would comprise an aqueous carboxylic acid mixture which when recycled to the liquid/liquid extraction zone would tend to build water in the system, and decrease the efficiency of reconcentration of sulfuric acid as discussed above. Since low selectivity for water absorbtion by the carboxylic acid solvent in the liquid/liquid extraction zone 160 is critical, the introduction of steam by a steam distillation or a steam striping process is

not preferred. Also, it has been found that alcohol-water-carboxylic acid extraction solvent mixtures can present settling problems in recovering alcohol and/or the carboxylic acid solvent therefrom, when such steam distillation methods are attempted.

The manner in which the acid/alcohol feed¸ stream 104 is formed is not critical to this invention. When feedstream 104 is formed by absorption of olefin into a strong acid, any of the prior art methods and absorption apparatus can be used, as described above.

Therefore, in one embodiment of the process of this invention, feedstream 104 can be formed by passing an olefin stream 102 to conventional absorbing zone 110 for countercurrent contact therein with a concentrated strong acid stream 106. Olefin 102 can comprise any of the above-mentioned aliphatic olefins having from 3 to 8 carbon atoms per molecule, and particularly olefins have 3 or 4 carbon atoms per molecule. Similarly, the identity and concentration of the strong acid in the acid stream 106, the temperature and pressure and other conditions used in absorbing zone 110 correspond to those which have been discussed earlier. Therefore, when the olefin comprises propylene, and the strong acid is sulfuric acid, stream 106 will generally comprise sulfuric acid of an acid strength of from about 45 to 99%, more preferably from about 50 to 80%, and when the olefin 102 comprises butylene or higher olefin feeds, stream 106 will generally comprise sulfuric acid of an acid strength of from about 45 to 85 wt.% and more preferably from about 55 to 80 wt.%. Generally, a temperature of from about 20 to 150° C and a pressure of from about 60 to 500 psig will be used.

Further, any of the above-discussed strong acids can be employed, and such acids will generally comprise a strong organic or inorganic acid which is miscible with water and which is characterized by dissociation constants ("pK" values) in aqueous solutions of less than about 3.5. Examples of suitable inorganic olefin hydration acids are hydrofluoric acid, hydroiodic acid, hydrochloric acid, ortho-phosphoric acid, phosphorous acid, perchloric acid, sulfuric acid and the like. Sulfuric acid is especially preferred. Examples of suitable organic olefin hydration acids are chloroacetic acid, benzene sulfonic acid and the like.

Unabsorbed gases are withdrawn from the upper portion of absorbing zone 110 via conduit 107 and can be recycled if desired to conduit 102 or subjected to conventional scrubbing/washing treatment, as with caustic solutions.

Therefore, the alcohols recovered by the process of this invention comprise the alcohol corresponding to the olefin(s) fed to the absorbing zone 110, and generally comprise saturated mono-alcohols having from 3 to 8 carbon atoms per molecule, and preferably having 3 or 4 carbon atoms per molecule. Examples of such alcohols are n-butanol, iso-butanol, sec-butanol, tert-butyl alcohol, n-propanol, iso-propanol, pentanols, hexanols and octanols.

As used herein, the term "extract saturation" (i.e., "E.S." values) of strong acid solutions, containing alcohol and/or alkyl ester of the strong acid, is defined by the expression (III):

$$E.S. = \frac{X^1}{X^A} \qquad (III)$$

wherein $X^1$ is the mole fraction of alcohol (and alcohol-equivalents represented by the alkyl esters) absorbed in the liquid and $X^A$ is the mole fraction in the liquid of the strong acid and strong acid moieties of the strong acid esters.

In the Examples, sulfuric acid concentrations are expressed as "acid strength" i.e., "A.S." values (defined above), unless otherwise indicated.

EXAMPLE 1

Formation of Aqueous Strong Acid Feed

A mixture of butene, secondary butyl ether ("SBE"), sec-butyl alcohol ("SBOH"), butyl ester of sulfuric acid ("$BuHSO_4$") sulfuric acid, and water was prepared in the selected amounts and passed as an acid/alcohol feedstream (having an acid strength of 55 wt.% $H_2SO_4$ and an extract saturation value of 1.0) to the upper portion of a continuous Karr extraction unit comprising a reciprocating plate column (total liquid holdup equals 650 cc.) and contacted therein with a selected amount of neo-decanoic acid which was passed to the reciprocating plate column at the lower portion thereof. The liquids thus passed to the extraction unit were preheated each to a temperature of 60° C. The reciprocating plate column was provided

with a hot water heating jacket maintained at 60 ° C. The upper portion of the extraction unit column was provided with a phase separator whereby a "light phase", comprising the neodecanoic acid and extracted sec-butyl alcohol values, were accumulated and withdrawn for analysis. From the bottom portion of the column, a "heavy phase", comprising the alcohol depleted sulfuric acid stream, was also withdrawn and collected, for analysis. The liquids thereby accumulated were each analyzed by gas chromatography, wet chemical methods and nuclear magnetic resonance spectroscopy (NMR) for component identification. The heavy phase was found to have a 60-percent acid strength (compared to the 55-percent acid strength of the feedstream to the extraction column) and an extract saturation value of 0.5 and was found to contain 0.1 wt.% neo-decanoic acid. (Secondary-butyl ester of neo-decanoic acid was also present in the neo-decanoic acid fed to, and recovered from, the process, in a weight:weight ratio of such secondary-butyl ester:neo-decanoic acid of about 0.03:1).

The data thereby obtained are set forth in Table I.

**TABLE 1: Continuous Extraction[1]**
**Solvent = neo-decanoic acid**

| Components | Acid/Alcohol Feedstream[2] Flow Rate | | Solvent[3] Flow Rate | | Light Phase Flow Rate | | Heavy Phase Flow Rate | | Overall Partition Coefficients |
|---|---|---|---|---|---|---|---|---|---|
| | GMS | Moles | GMS | Moles | GMS | Moles | GMS | Moles | Wt. Fract. (4) |
| Butene | 0.268 | 0.005 | 0 | 0 | 1.38 | 0.025 | 0.011 | 0.0001 | 43.88 |
| SBE | 0.011 | ~0 | 0 | 0 | 0.128 | 0.001 | 0.0035 | ~0 | 12.79 |
| SBOH | 34.35 | 0.464 | 0 | 0 | 25.62 | 0.346 | 7.113 | 0.096 | 1.26 |
| $BuHSO_4$ | 1.138 | 0.007 | 0 | 0 | 0 | 0 | 0.073 | 0.0004 | 0 |
| $H_2SO_4$ | 45.97 | 0.469 | 0 | 0 | 0.051 | 0.0005 | 46.60 | 0.475 | 0.0004 |
| Water | 29.86 | 1.659 | 0.068 | 0.004 | 1.100 | 0.0611 | 29.21 | 1.623 | 0.0132 |
| Solvent | 0 | 0 | 227.7 | 1.324 | 227.6 | 1.323 | 0.109 | 0.0006 | 730.3 |
| Total | 111.6 | 2.605 | 227.8 | 1.328 | 255.9 | 1.757 | 89.5 | 2.196 | ----- |

(1) Column residence time = 3.52 hours. Solvent-to-feed weight ratio = 2.04:1. Flow rates expressed as units/hour.
(2) Feed: E.S.=1; A.S.=55%; specific gravity, 25 °C=1.21; Temp.=60°C
(3) Solvent = neo-decanoic acid (Exxon Chemical Americas, >95% purity)
(4) Calculated from component's concentration in light phase ÷ its concentration in heavy phase.

EXAMPLE 2

The heavy phase, comprising an aqueous strong acid raffinate formed as in Example 1 was then contacted in a series of runs with an octene, i.e., 2,4,4-trimethyl-1-pentene, in varying proportions, by charging the selected amount of the aqueous strong raffinate to a 60 cc standard laboratory separatory funnel. After addition of the selected amount the 2,4,4-trimethyl-1-pentene, the liquids were agitated and

then allowed to settle for 60 minutes at 25 °C under atmospheric conditions. At the end of this time, each liquid phase was recovered and analyzed to determine the weights of the upper and lower liquid layers and the wt.% content of neo-decanoic acid in each. The data thereby obtained are set forth in Table II below.

As can be seen from the data in Table II, the liquid organic solvent effected a rapid extraction of the neo-decanoic acid from the aqueous strong-acid phase, and permitted recovery of a treated aqueous strong-acid phase having significantly depleted concentrations of the neo-acid.

TABLE II

| Run No. | Charged: Aq. Raffinate: $Me_3$ pentene (vol:vol) | Charged: Aq. Raffinate wt (gms) | Charged: $Me_3$ Pentene wt (gms) | Recovered: Upper Layer (gms) | Recovered: Lower(1) Layer (gms) | Recovered: [NDA] in Upper Layer (wt%) | Recovered: [NDA] in Lower Layer (wt%) | % NDA Removal From Charge |
|---|---|---|---|---|---|---|---|---|
| 2-0 (ref) | | | | | | | 1.00 | |
| 2-1 | 20/1 | 25.4515 | 0.8018 | 0.9612 | 25.2911 | 22.30 | 0.12 | 88 |
| 2-2 | 15/1 | 18.4636 | 0.8200 | 1.0922 | 18.1914 | 18.06 | 0.12 | 88 |
| 2-3 | 10/1 | 12.7922 | 0.8251 | 0.9375 | 12.6798 | 13.03 | 0.08 | 92 |
| 2-4 | 5/1 | 13.0050 | 1.6189 | 1.7509 | 12.8730 | 6.95 | 0.08 | 92 |
| 2-5 | 3/1 | 19.0728 | 3.5350 | 3.7170 | 18.8908 | 4.87 | 0.05 | 95 |

Note: "$Me_3$ Pentene" = (2,4,4 trimethyl 1-pentene). NDA = neo-decanoic acid.

(1) Gms. lower layer = (total gms. of charge - gms. upper layer).

EXAMPLE 3

The procedure in Example 2 was repeated employing di-(secondary-butyl) ether ("SBE") as the liquid organic solvent, in the volumes indicated in Table III. The data thereby obtained are set forth in Table III.

TABLE III

| Run No. | Charged | | | Recovered | | | | % NDA Removed From Charge |
|---|---|---|---|---|---|---|---|---|
| | Aq. Raffinate: SBE (vol:vol) | Aq. Raffinate wt (gms) | SBE wt (gms) | Upper Layer (gms) | Lower(1) Layer (gms) | [NDA] in Upper Layer (wt%) | [NDA] in Lower Layer (wt%) | |
| 3-1 | 10/1 | 13.1345 | 0.7606 | 0.1943 | 13.7006 | 38.49 | 0.41 | 57 |
| 3-2 | 10/2 | 12.9158 | 1.5187 | 0.5976 | 13.8369 | 14.18 | 0.26 | 72 |
| 3-3 | 15/5 | 19.2635 | 3.7304 | 2.1340 | 20.8599 | 7.67 | 0.12 | 87 |

Note: "SBE" = di(secondary butyl) ether. NDA = neo-decanoic acid.

(1) Gms. lower layer = (total gms. of charge - gms. upper layer).

EXAMPLE 4

The procedure used in Example 2 was repeated except that a temperature of 60 ° C was used to contact the liquids during settling and except that heavy phase comprised an aqueous sulfuric acid raffinate which was formed by the following procedure: A sulfuric acid-alcohol mixture comprising 15.6 wt.% secondary butyl alcohol, 16.4 wt.% butyl ester sulfuric acid (calculated as $C_4H_9HSO_4$, 48.5 wt.% sulfuric acid (calculated as 100% $H_2SO_4$), 17.1 wt.% water and minor amounts of secondary butyl ether and $C_5$ alcohols, together with about 0.2 wt.% surfactants (primarily butyl hydroxy sulfonic acid), mixing 30 cc of the sulfuric acid-alcohol mixture with about 4 cc neodecanoic acid and 4 cc of secondary butyl neodecanoate, for extraction of at least a portion of the alcohol by the mixture of the neodecanoic acid in the secondary butyl neodecanoate extraction solvent. The resulting two-phase liquid mixture was shaken well for two minutes in a separatory funnel, and the liquids were allowed to separate by settling for 15 minutes at room temperature and atmospheric pressure. The liquid phases were then recovered and the lower liquid phase, comprising the aqueous sulfuric acid raffinate, from which alcohol was extracted and which contained about 0.60 wt.% neodecanoic acid and about 0.80 wt.% secondary butyl neodecanoate was used as the heavy phase for further treatment to remove the neodecanoic acid and ester therefrom. The data thereby obtained in contacting the above-described heavy phase with octene solvent is set forth in Table IV below.

TABLE IV

| Run No. | Charged: Aq. Raffinate: $Me_3$ pentene (vol:vol) | Charged: Aq. Raffinate wt (gms) | Charged: $Me_3$ pentene wt (gms) | Recovered: Upper Layer (gms) | Recovered: Lower(1) Layer (gms) | Recovered: [NDA] in Upper Layer (wt%) | Recovered: [NDA] in Lower Layer (wt%) | Recovered: [$C_4$NDA Ester] in Upper Layer (wt.%) | Recovered: [$C_4$NDA Ester] in Lower Layer (wt.%) |
|---|---|---|---|---|---|---|---|---|---|
| 4-1 | 10/1 | 13.0309 | 0.7173 | 0.9691 | 12.7791 | 7.91 | 0.01 | 10.37 | 0.02 |
| 4-2 | 10/2 | 13.2954 | 1.4436 | 1.7518 | 12.9872 | 4.39 | 0.01 | 5.89 | 0.02 |

Note: "Me 3 Pentene" = (2,4,4 trimethyl 1-pentene). NDA = neo-decanoic acid. "$C_4$ NDA Ester" = sec-butyl neodecanoate.

(1) Gms. lower layer = (total gms. of charge - gms. upper layer).

EXAMPLE 5

The procedure of Example 4 was repeated except that the organic extractive agent added was di-(secondary-butyl) ether ("SBA") and except that the aqueous sulfuric acid raffinate so prepared, for use in

this experiment contained 0.844 wt.% neo-decanoic acid and 0.603 wt.% sec-butyl neodecanoate. The data thereby obtained are summarized in Table V below.

TABLE V

| Run No. | Charged | | | Recovered | | | | | |
|---|---|---|---|---|---|---|---|---|---|
| | Aq. Raffinate: SBE (vol:vol) | Aq. Raffinate wt (gms) | SBE wt (gms) | Upper Layer (gms) | Lower(1) Layer (gms) | [NDA] in Upper Layer (wt%) | [NDA] in Lower Layer (wt.%) | [$C_4$NDA Ester] in Upper Layer (wt.%) | [$C_4$NDA Ester] in Lower Layer (wt.%) |
| 5-1 | 10/1 | 12.9242 | 0.7696 | 0.6660 | 13.0278 | 15.46 | 0.03 | 13.30 | 0.14 |
| 5-2 | 10/2 | 12.9362 | 1.5210 | 1.2870 | 13.1702 | 5.8 | 0.045 | 4.61 | 0.02 |

Note: "SBE" = di(secondary butyl) ether. NDA = neo-decanoic acid. "$C_4$ NDA Ester" = sec-butyl-neodecanoate.

(1) Gms. lower layer = (total gms. of charge - gms. upper layer).

EXAMPLE 6

The procedure of Example 4 was repeated except that the organic extractive agent comprised hexene-1. The data thereby obtained are summarized in Table VI.

Table VI

| Run No. | Charged: Aq. Raffinate Hexene-1 (vol:vol) | Recovered: [NDA] in Upper Layer (wt.%) | Recovered: [NDA] in Lower Layer (wt.%) | Recovered: [C4 NDA Ester] in Upper Layer (wt.%) | Recovered: [C4 NDA Ester] in Lower Layer (wt.%) |
|---|---|---|---|---|---|
| 6-1 | 10/1 | 7.165 | 0.025 | 6.123 | 0.025 |
| 6-2 | 10/2 | 3.685 | 0.004 | 3.600 | 0.004 |

Notes: "SBE" = di(secondary butyl) ether. NDA = neo-decanoic acid. "$C_4$ NDA Ester" = sec-butyl neodecanoate.

(1) Gms. lower layer = (total gms. of charge - gms. upper layer).

EXAMPLE 7

120 cc of a sulfuric acid-alcohol mixture containing neo-decanoic acid and sec-butyl neodecanoate, prepared as in Example 4 is charged into a 150 cc Hoke gas cylinder equipped with a valve at each end. Cis-butene-2 was then introduced into the first cylinder from a second such 150 cc Hoke gas cylinder, containing 14.4 gm (24 cc) of liquid cis-butene-2. The first cylinder was then sealed and shaken for 5 minutes to mix the liquids therein. Thereafter, the first cylinder was placed upright in a water bath (60 ° C),

again shaken after 10 minutes of settling time, and then left in the water bath for 2 hours. At the end of this time, the liquid phases were carefully separated for analysis. The data thereby obtained are set forth in Table VII.

Table VII

| Run No. | Charged Aq. Raffinate Butene (vol:vol) | Recovered [NDA] in Upper Layer (wt.%) | Recovered [NDA] in Lower Layer (wt.%) | Recovered [C4 NDA Ester] in Upper Layer (wt.%) | Recovered [C4 NDA Ester] in Lower Layer (wt.%) |
|---|---|---|---|---|---|
| 7-1 | 10/1 | 6.52 | 0.01 | 6.44 | 0.02 |

Notes: NDA = neo-decanoic acid. "$C_4$ NDA Ester" = *sec*-butyl neodecanoate.

(1) Gms. lower layer = (total gms. of charge - gms. upper layer).

It will be understood from the foregoing that while the alcohol-recovery method of this invention has been particularly described with regard to alcohol recovery from acid/alcohol feedstreams produced by absorbing olefins into concentrated strong acids, the method of this invention may be employed for recovery of alcohol from such acid/alcohol mixtures produced by different processes, either as product or by-product streams.

It will be obvious that various changes and modifications may be made without departing from the invention and it is intended, therefore, that all matter contained in the foregoing description shall be interpreted as illustrative only and not limitative of the invention.

## Claims

**1.** A process for recovering an aliphatic mono-alcohol having from 3 to 8 carbon atoms per molecule from an acid/alcohol feedstream comprising an aqueous solution of a strong acid of 40 to 80% acid strength containing said alcohol which comprises:

(a) contacting said feedstream in an extraction zone with an alicyclic and/or acyclic carboxylic acid having from 6 to 20 carbon atoms per molecule and/or mixtures thereof to form a first liquid phase comprising an alcohol-enriched carboxylic acid extract, and a second liquid phase comprising an aqueous strong acid raffinate depleted in alcohol and containing from 0.1 to 2 wt.% of said carboxylic acid extraction solvent,

(b) recovering said alcohol-enriched carboxylic acid extract, and

(c) contacting said second liquid phase with from 5 to 60 parts by weight per 100 parts of said second liquid phase of an organic extractive agent to separate at least a majority of said carboxylic acid extraction solvent from said second liquid phase and to form a third liquid phase comprising said separated carboxylic acid extraction solvent and said organic extractive agent, and a fourth liquid phase comprising an aqueous strong acid solution depleted in alcohol and containing less than 1 wt.% of said carboxylic acid extraction solvent.

**2.** The process of claim 1 wherein said strong acid comprises sulphuric acid.

**3.** The process of claim 1 or claim 2 wherein said organic extractive agent comprises a $C_3$ to $C_{12}$ alkene or a $C_6$ to $C_{20}$ dialkyl ether.

**4.** The process of any one of the preceding claims including the steps of separating the components of said third liquid phase, recycling said carboxylic acid extraction solvent to step (a) and recycling said organic extractive agent to step (c).

**5.** The process of any one of the preceding claims wherein said acid/alcohol feedstream is obtained by the step of absorbing an alkene corresponding to said alcohol in a said strong acid solution under conditions sufficient to form an alkyl ester of said strong acid of which the alkyl group corresponds to said alkene and said alcohol, the process including the step of recycling said fourth liquid phase to said absorbing step.

**6.** The process of claim 5 wherein said organic extractive agent is related to the alcohol produced in said absorbing step as follows:-

| Alcohol | Organic extractive agent |
|---|---|
| isopropyl | propylene or diisopropyl ether |
| sec. butyl | n-butenes or sec.butyl ether |
| tert.butyl | isobutylene or di-tert-butyl ether |

**7.** The process of claim 5 or claim 6 wherein said organic extractive agent is an alkene corresponding to that used in said absorbing step and said process includes the steps of separating the components of said third liquid phase and recycling said organic extractive agent to said absorbing step.

**8.** The process of any one of claims 5 to 7 wherein said acid/alcohol stream also contains an alkyl ester of said strong acid, wherein said alkyl moiety corresponds to said alcohol, and wherein said acid/alcohol feedstream is admixed with from about 0.04 to 0.4 parts by weight of water per part by weight of said acid/alcohol feedstream prior to said extraction zone.

**9.** The process of any one of the preceding claims wherein said acid/alcohol feedstream and said carboxylic acid extraction solvent are contacted in a continuous countercurrent extraction zone.

**10.** The process of claim 9 wherein said carboxylic acid extraction solvent comprises the continuous phase and said acid/alcohol feedstream comprises the discontinuous phase in said extraction zone.

**11.** The process of any one of the preceding claims wherein said alcohol-enriched carboxylic acid extract contains less than 1 wt. % of said strong acid.

**12.** The process of any one of the preceding claims wherein said alcohol-enriched carboxylic acid extract contains less than 2 wt. % water.

**13.** The process of any one of the preceding claims wherein said aqueous strong acid raffinate depleted in alcohol contains said strong acid in an acid strength of at least 1 wt. % greater than the acid strength of said strong acid in said acid/alcohol feedstream introduced to said extraction zone.

**14.** The process of claim 13 wherein said strong acid strength in said raffinate is from 2 to 7 wt. % greater than the acid acid strength of said acid/alcohol feedstream introduced to said extraction zone.

**15.** The process of any one of the preceding claims wherein said alcohol-enriched carboxylic acid extract contains said alcohol in a concentration of from 5 to 20 wt. %.

**16.** The process of any one of the preceding claims wherein said carboxylic acid extraction solvent comprises at least one acid of the formula: $RCO_2H$, where R is an alicyclic or acyclic alkyl group having from 5 to 19 carbon atoms.

**17.** The process of any of claims 1 to 15 wherein said carboxylic acid extraction solvent comprises a neo-acid having from 6 to 20 carbons per molecule and mixtures thereof.

**18.** The process of claim 17 wherein said neo acid is selected from the group consisting of acids of the formula

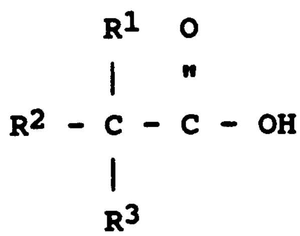

wherein $R^1$, $R^2$ and $R^3$ are the same or different and are independently selected from the group consisting of alkyl of from 1 to 16 carbon atoms, with the proviso that the neo acid contains a total of from 6 to 20 carbon atoms per molecule.

**19.** The process of claim 18 wherein $R^1$ and $R^2$ are each alkyl groups having from 1 to 3 carbon atoms per alkyl group, and $R^3$ is alkyl of from 1 to 6 carbon atoms.

**20.** The process of any one of the preceding claims wherein said carboxylic acid extraction solvent is employed in said extraction zone in an amount of from about 0.3 to 5 parts by weight of said acid/alcohol feedstream.

**Revendications**

**1.** Procédé pour récupérer un mono-alcool aliphatique ayant 3 à 8 atomes de carbone par molécule d'un courant de charge acide/alcool comprenant une solution aqueuse d'un acide fort ayant une force d'acide de 40 à 80 % comprenant ledit alcool, qui consiste :

(a) à faire entrer ledit courant de charge en contact dans une zone d'extraction avec un acide carboxylique alicyclique et/ou acyclique ayant 6 à 20 atomes de carbone par molécule et/ou des mélanges de tels acides pour former une première phase liquide comprenant un extrait carboxylique enrichi en alcool et une seconde phase liquide comprenant une phase aqueuse raffinée d'acide fort épuisée en alcool et contenant 0,1 à 2 % en poids dudit acide carboxylique utilisé comme solvant d'extraction,

(b) à recueillir ledit extrait carboxylique enrichi en alcool et

(c) à faire entrer la seconde phase liquide en contact avec 5 à 60 parties en poids pour 100 parties en poids de ladite seconde phase liquide d'un agent organique d'extraction pour séparer au moins la majeure partie dudit solvant carboxylique d'extraction de la seconde phase liquide et pour former une troisième phase liquide comprenant ledit solvant carboxylique d'extraction séparé et ledit agent organique d'extraction et une quatrième phase liquide comprenant une solution aqueuse d'acide fort épuisée en alcool et contenant moins de 1 % en poids dudit acide carboxylique utilisé comme

solvant d'extraction.

**2.** Procédé suivant la revendication 1, dans lequel l'acide fort comprend de l'acide sulfurique.

**3.** Procédé suivant la revendication 1 ou la revendication 2, dans lequel l'agent organique d'extraction comprend un alcène en $C_3$ à $C_{12}$ ou un éther de dialkyle en $C_6$ à $C_{20}$.

**4.** Procédé suivant l'une quelconque des revendications précédentes, qui comprend les étapes de séparation des composants de ladite troisième phase liquide, recyclage du solvant carboxylique d'extraction dans l'étape (a) et recyclage de l'agent organique d'extraction dans l'étape (c).

**5.** Procédé suivant l'une quelconque des revendications précédentes, dans lequel ledit courant de charge acide/alcool est obtenu par l'étape d'absorption d'un alcène correspondant audit alcool dans une solution d'acide fort dans des conditions suffisantes pour former un ester alkylique de l'acide fort dont le groupe alkyle correspond à l'alcène et à l'alcool, le procédé comprenant l'étape de recyclage de la quatrième phase liquide dans l'étape d'absorption.

**6.** Procédé suivant la revendication 5, dans lequel l'agent organique d'extraction est en relation avec l'alcool produit dans ladite étape d'absorption comme suit :

| Alcool | Agent organique d'extraction |
|---|---|
| Isopropylique | Propylène ou éther de diisopropyle |
| Sec.-butylique | n-butènes ou éther de sec.-butyle |
| Tertio-butylique | Isobutylène ou éther de ditertio-butyle |

**7.** Procédé suivant la revendication 5 ou la revendication 6, dans lequel l'agent organique d'extraction est un alcène correspondant à celui qui est utilisé dans l'étape d'absorption et le procédé comprend les étapes de séparation des composants de la troisième phase liquide et de recyclage dudit agent organique d'extraction dans ladite étape d'absorption.

**8.** Procédé suivant l'une quelconque des revendications 5 à 7, dans lequel le courant acide/alcool contient aussi un ester alkylique dudit acide fort, le groupement alkyle correspond audit alcool et le courant de charge acide/alcool est mélangé avec environ 0,04 à 0,4 partie en poids d'eau par partie en poids dudit courant de charge acide/alcool avant ladite zone d'extraction.

**9.** Procédé suivant l'une quelconque des revendications précédentes, dans lequel le courant de charge acide/alcool et l'acide carboxylique utilisé comme solvant d'extraction sont mis en contact dans une zone d'extraction continue à contre-courant.

**10.** Procédé suivant la revendication 9, dans lequel l'acide carboxylique utilisé comme solvant d'extraction constitue la phase continue et le courant de charge acide/alcool constitue la phase discontinue dans ladite zone d'extraction.

**11.** Procédé suivant l'une quelconque des revendications précédentes, dans lequel l'extrait carboxylique enrichi en alcool contient moins de 1 % en poids dudit acide fort.

**12.** Procédé suivant l'une quelconque des revendications précédentes, dans lequel l'extrait carboxylique enrichi en alcool contient moins de 2 % en poids d'eau.

**13.** Procédé suivant l'une quelconque des revendications précédentes, dans lequel la phase aqueuse raffinée d'acide fort épuisée en alcool contient ledit acide fort avec une force d'acide supérieure d'au moins 1 % en poids à la force de l'acide fort dans ledit courant de charge acide/alcool introduit dans ladite zone d'extraction.

**14.** Procédé suivant la revendication 13, dans lequel la force de l'acide fort dans la phase raffinée est supérieure de 2 à 7 % en poids à la force d'acide dans ledit courant de charge acide/alcool introduit

dans la zone d'extraction.

**15.** Procédé suivant l'une quelconque des revendications précédentes, dans lequel l'extrait carboxylique enrichi en alcool contient ledit alcool à une concentration de 5 à 20 % en poids.

**16.** Procédé suivant l'une quelconque des revendications précédentes, dans lequel le solvant carboxylique d'extraction comprend au moins un acide de formule $RCO_2H$ dans laquelle R est un groupe alkyle alicyclique ou acyclique ayant 5 à 19 atomes de carbone.

**17.** Procédé suivant l'une quelconque des revendications 1 à 15, dans lequel l'acide carboxylique utilisé comme solvant d'extraction comprend un néo-acide ayant 6 à 20 atomes de carbone par molécule et des mélanges de ces acides.

**18.** Procédé suivant la revendication 17, dans lequel le néo-acide est choisi dans le groupe comprenant des acides de formule

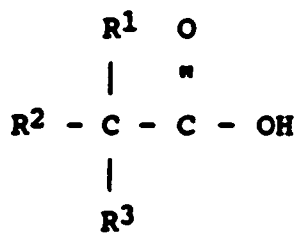

dans laquelle $R^1$, $R^2$ et $R^3$ sont identiques ou différents et sont choisis indépendamment dans le groupe comprenant des radicaux alkyle ayant 1 à 16 atomes de carbone, sous réserve que le néo-acide contienne un total de 6 à 20 atomes de carbone par molécule.

**19.** Procédé suivant la revendication 18, dans lequel $R^1$ et $R^2$ sont tous deux des groupes alkyle ayant 1 à 3 atomes de carbone chacun et $R^3$ est un groupe alkyle ayant 1 à 6 atomes de carbone.

**20.** Procédé suivant l'une quelconque des revendications précédentes, dans lequel l'acide carboxylique servant de solvant d'extraction est utilisé dans ladite zone d'extraction en une quantité d'environ 0,3 à 5 parties en poids dudit courant de charge acide/alcool.

**Patentansprüche**

**1.** Verfahren zur Gewinnung von aliphatischem Monoalkohol mit 3 bis 8 Kohlenstoffatomen pro Molekül aus einem Säure/Alkohol-Einsatzmaterialstrom,der eine den Alkohol enthaltende wäßrige Lösung einer starken Säure mit einer Säurestärke von 40 bis 80% umfaßt, bei dem:

a) der Einsatzmaterialstrom in einer Extraktionszone mit einer alicyclischen und/oder acyclischen Carbonsäure mit 6 bis 20 Kohlenstoffatomen pro Molekül und/oder Mischungen derselben kontaktiert wird, um eine erste flüssige Phase, die einen mit Alkohol angereicherten Carbonsäureextrakt umfaßt, und eine zweite flüssige Phase zu bilden, die ein an Alkohol verarmtes, wäßriges, stark saures Raffinat umfaßt und 0,1 bis 2 Gew.% des Carbonsäureextraktionslösungsmittels enthält,

b) der mit Alkohol angereicherte Carbonsäureextrakt gewonnen wird und

c) die zweite flüssige Phase mit 5 bis 60 Gewichtsteilen eines organischen Extraktionsmittels pro 100 Gewichtsteile der zweiten flüssigen Phase kontaktiert wird, um mindestens einen Großteil des Carbonsäureextraktionslösungsmittels von der zweiten flüssigen Phase zu trennen, eine dritte flüssige Phase, die das abgetrennte Carbonsäureextraktionslösungsmittel und das organische Extraktionsmittel umfaßt, und eine vierte flüssige Phase zu bilden, die eine an Alkohol verarmte, wäßrige, stark saure Lösung umfaßt und weniger als 1 Gew.% des Carbonsäureextraktionslösungsmittels enthält.

**2.** Verfahren nach Anspruch 1, bei dem die starke Säure Schwefelsäure umfaßt.

**3.** Verfahren nach Anspruch 1 oder Anspruch 2, bei dem das organische Extraktionsmittel ein $C_3$-$C_{12}$-

Alken oder einen $C_6$-$C_{20}$-Dialkylether umfaßt.

4. Verfahren nach einem der vorhergehenden Ansprüche, bei dem die Komponenten der dritten flüssigen Phase getrennt werden, das Carbonsäureextraktionslösungsmittel zu Schritt (a) zurückgeführt wird und das organische Extraktionsmittel zu Schritt (c) zurückgeführt wird.

5. Verfahren nach einem der vorhergehenden Ansprüche, bei dem der Säure/Alkohol-Einsatzmaterialstrom erhalten wird, indem ein mit dem Alkohol korrespondierendes Alken in einer stark sauren Lösung unter Bedingungen adsorbiert wird, die ausreichen, um einen Alkylester der starken Säure zu bilden, dessen Alkylgruppe mit dem Alken und dem Alkohol korrespondiert, und die vierte flüssige Phase zu dem Absorptionsschritt zurückgeführt wird.

6. Verfahren nach Anspruch 5, bei dem das organische Extraktionsmittel mit dem in dem Absorptionsschritt hergestellten Alkohol wie folgt in Bezug steht:

| Alkohol | Organisches Extraktionsmittel |
|---|---|
| Isopropyl-<br>sek. Butyl-<br>tert. Butyl- | Propylen oder Diisopropylether<br>n-Butene oder sek. Butylether<br>Isobutylen oder Di-tert.-butylether |

7. Verfahren nach Anspruch 5 oder Anspruch 6, bei dem das organische Extraktionsmittel ein Alken ist, das mit dem im Absorptionsschritt verwendeten korrespondiert, die Komponenten der dritten flüssigen Phase getrennt werden und das organische Extraktionsmittel zu dem Absorptionsschritt zurückgeführt wird.

8. Verfahren nach einem der Ansprüche 5 bis 7, bei dem der Säure/Alkohol-Strom weiterhin einen Alkylester der starken Säure enthält, wobei der Alkylrest mit dem Alkohol korrespondiert, und bei dem der Säure/Alkohol-Einsatzmaterialstrom vor der Extraktionszone mit etwa 0,04 bis 0,4 Gewichtsteilen Wasser pro Gewichtsteil des Säure/Alkohol-Einsatzmaterialstroms gemischt wird.

9. Verfahren nach einem der vorhergehenden Ansprüche, bei dem der Säure/Alkohol-Einsatzmaterialstrom und das Carbonsäureextraktionslösungsmittel in einer kontinuierlichen Gegenstromextraktionszone kontaktiert werden.

10. Verfahren nach Anspruch 9, bei dem in der Extraktionszone das Carbonsäureextraktionslösungsmittel die kontinuierliche Phase umfaßt und der Säure/Alkohol-Einsatzmaterialstrom die diskontinuierliche Phase umfaßt.

11. Verfahren nach einem der vorhergehenden Ansprüche, bei dem der mit der Alkohol angereicherte Carbonsäureextrakt weniger als 1 Gew.% der starken Säure enthält.

12. Verfahren nach einem der vorhergehenden Ansprüche, bei dem der mit Alkohol angereicherte Carbonsäurextrakt weniger als 2 Gew.% Wasser enthält.

13. Verfahren nach einem der vorhergehenden Ansprüche, bei dem das an Alkohol verarmte, wäßrige, stark saure Raffinat die starke Säure in einer Säurestärke enthält, die mindestens 1 Gew.% größer als die Säurestärke der starken Säure in dem der Extraktionszone zugeführten Säure/Alkohol-Einsatzmaterialstrom ist.

14. Verfahren nach Anspruch 13, bei dem die Stärke der starken Säure in dem Raffinat 2 bis 7 Gew.% größer als die Säurestärke des der Extraktionszone zugeführten Säure/Alkohol-Einsatzmaterialstroms ist.

15. Verfahren nach einem der vorhergehenden Ansprüche, bei dem der mit Alkohol angereicherte Carbonsäureextrakt den Alkohol in einer Konzentration von 5 bis 20 Gew.% enthält.

**16.** Verfahren nach einem der vorhergehenden Ansprüche, bei dem das Carbonsäureextraktionslösungsmittel mindestens eine Säure mit der Formel: $RCO_2H$ umfaßt, in der R eine alicyclische oder acyclische Alkylgruppe mit 5 bis 19 Kohlenstoffatomen ist.

**17.** Verfahren nach einem der Ansprüche 1 bis 15, bei dem das Carbonsäureextraktionslösungsmittel eine Neo-Säure mit 6 bis 20 Kohlenstoffatomen pro Molekül und Mischungen derselben umfaßt.

**18.** Verfahren nach Anspruch 17, bei dem die Neo-Säure ausgewählt ist aus der Gruppe bestehend aus Säuren mit der Formel:

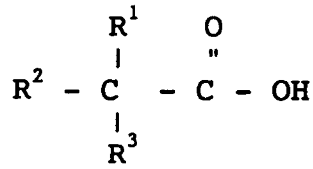

in der $R^1$, $R^2$ und $R^3$ gleich oder verschieden sind und unabhängig voneinander ausgewählt sind aus der Gruppe bestehend aus Alkylgruppen mit 1 bis 16 Kohlenstoffatomen, mit der Maßgabe, daß die Neo-Säure insgesamt 6 bis 20 Kohlenstoffatome pro Molekül enthält.

**19.** Verfahren nach Anspruch 18, bei dem $R^1$ und $R^2$ jeweils Alkylgruppen mit 1 bis 3 Kohlenstoffatomen pro Alkylgruppe sind und $R^3$ eine Alkylgruppe mit 1 bis 6 Kohlenstoffatomen ist.

**20.** Verfahren nach einem der vorhergehenden Ansprüche, bei dem das Carbonsäureextraktionslösungsmittel in der Extraktionszone in einer Menge von etwa 0,3 bis 5 Gewichtsteilen des Säure/Alkohol-Einsatzmaterialstroms verwendet wird.

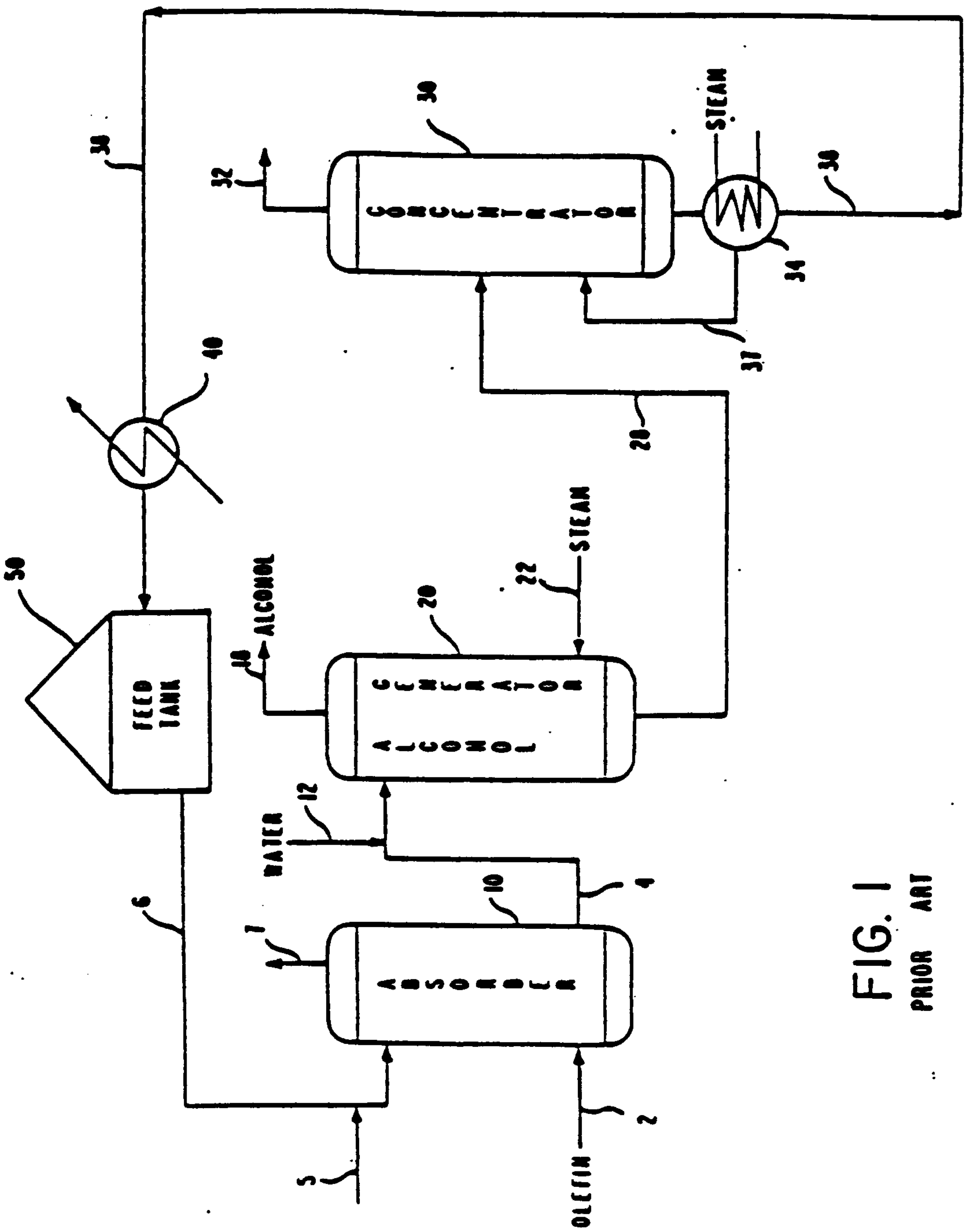


FIG. 1
PRIOR ART

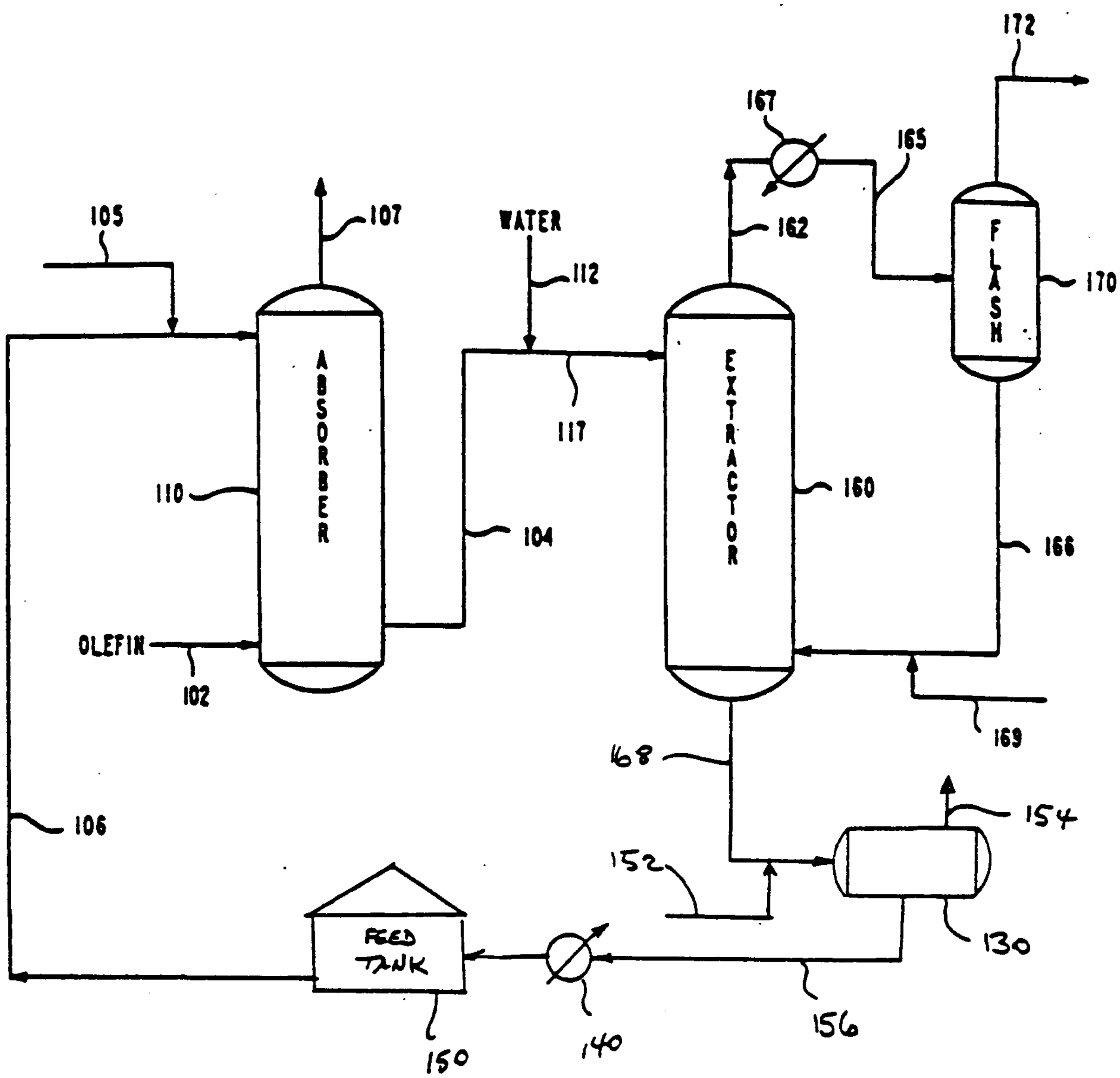
172
167
165
105
107
WATER
112
162
FLASH
170
ABSORBER
117
EXTRACTOR
110
104
160
166
OLEFIN
102
169
168
154
106
152
FEED TANK
130
156
150
140

FIG. 2.